# EUROPEAN PATENT APPLICATION

(11) **EP 4 574 808 A1**
(43) Date of publication of application: **25.06.2025**
(21) Application number: 24161777.8
(22) Date of filing: 06.03.2024
(51) Int. Cl.: C07C 45/50, C07C 47/02

(54) **PROCESS FOR THE HYDROFORMYLATION OF AN ALIPHATIC ALKENE**

(30) Priority: 21.12.2023 FR 2314890
(71) Applicant: Université de Strasbourg, 67000 Strasbourg (FR); Centre National de la Recherche Scientifique, 75794 Paris Cedex 16 (FR)
(72) Inventor: DYDIO, Pawel, 67000 Strasbourg (FR); BOJANOWSKI, Jan, 67000 Strasbourg (FR)
(74) Representative: IP Trust

(57) **Abstract**

The invention relates to a method for the hydroformylation of an aliphatic alkene with carbon monoxide and hydrogen, the method implementing a catalyst which comprises Palladium (0) complex of a first ligand L1 and Cobalt (0), the first ligand L1 being a monodentate phosphine.

## Description

### FIELD OF THE INVENTION

The present invention concerns a method of hydroformylation of an aliphatic alkene under green conditions.

### TECHNOLOGICAL BACKGROUND OF THE INVENTION

Hydroformylation is a synthetic route to produce aldehydes by chemical reaction of an alkene with carbon monoxide CO and dihydrogen H₂ in the presence of a catalyst, typically a metal complex in combination with an alkyl or aryl phosphine. In particular, the hydroformylation of propylene is one of the most important reactions in the chemical industry. The catalysts generally used, which comprise Rh or Co, are not satisfactory.

In particular, the sourcing of Rh remains quite difficult.

In particular, the activity of the catalysts comprising Co as the sole catalyst remains either low or quickly decaying under green conditions, such as low pressures and low temperatures.

Furthermore, there is a need for developing catalysts which are compatible with green conditions and present a sufficiently high activity and/or a quite low activity decay.

Additionally, there is a need for reaction conditions that do not involve halogens forming acidic species during the process, leading to corrosive medium.

It is therefore an object of the present invention to provide a method for the hydroformylation of an aliphatic alkene involving a catalyst compatible with green conditions.

Another object of the present invention is to provide a method for the hydroformylation of an aliphatic alkene involving a catalyst having sufficiently high activity.

Another object of the present invention is to provide a method for the hydroformylation of an aliphatic alkene involving a catalyst having sufficiently low activity decay.

### BRIEF DESCRIPTION OF THE INVENTION

The present invention relates to a method for the hydroformylation of an aliphatic alkene with carbon monoxide CO and dihydrogen H₂, the method implementing a catalyst which comprises Palladium (0) complex of a first ligand L1 and Cobalt (0), the first ligand L1 being a monodentate phosphine.

According to one embodiment, the first ligand comprises at least one of the elements chosen among: tri-tert-butylphosphine of formula P(tBu)₃ (CAS : 13716-12-6), di(1-adamantyl)-n-butylphosphine, known as "cataCXium^{®}" (CAS : 321921-71-5), tert-butyldicyclohexylphosphine of formula P(tBu)Cy₂ (CAS : 93634-87-8), dicyclohexylphenylphosphine of formula PPhCy₂ (CAS : 6476-37-5), di-tert-butylphenylphosphine of formula PPh(tBu)₂ (CAS : 32673-25-9).

According to one embodiment, the Cobalt (0) forms a complex with a second ligand L2, said second ligand L2 being a bidentate phosphine.

According to one embodiment, the Cobalt (0) forms a complex with a second ligand L2, said second ligand L2 being a bidentate aliphatic phosphine.

According to one embodiment, the second ligand L2 comprises at least one of the elements chosen among: bis(dicyclohexylphosphino) methane (dcypm ; CAS : 137349-65-6), 1,2-bis(dicyclohexylphosphino) ethane (dcype ; CAS : 23743-26-2), 1,3-bis(dicyclohexylphosphino) propane (dcypp ; CAS : 103099-52-1), 1,4-bis(dicyclohexylphosphino) butane (dcypb ; CAS : 65038-36-0).

According to one embodiment, the molar ratio of palladium (0) over cobalt (0) is in the 1:10 to 10:1 range, advantageously in the 1:4 to 1:1 range.

According to one embodiment, the aliphatic alkene consists of propylene.

According to one embodiment, said method is carried out in a non-aqueous solvent which comprises at least one of the elements selected from: anisole, toluene, 1,4-dioxane, dimethylacetamide, p-xylene, decahydronaphthalene, DMF, DMSO, α,α,α-trifluorotoluene - trifluorotoluene, hexafluorobenzene, 1,2-dichlorobenzene, methyl benzoate.

According to one embodiment, said method is carried out in short-chain aldehydes as solvent.

By "short-chain aldehydes", it is meant aldehydes containing a carbon chain equal or inferior to 5. Advantageously, said method is carried out in butanal or isobutanal.

According to one embodiment, said method is carried out without any external solvent.

According to one embodiment, said method is carried out at a temperature of between 70 °C and 200 °C, advantageously in the 80 to 120 °C range.

According to one embodiment, carbon monoxide and hydrogen are provided at a pressure up to 50 bar each, preferably up to 10 bar each.

According to one embodiment, the aliphatic alkene is provided at a pressure up to 10 bar.

### BRIEF DESCRIPTION OF THE DRAWINGS

Further features and advantages of the invention will be apparent from the following detailed description with reference to the attached figures in which :
Figure 1 shows the chemical reaction associated with the hydroformylation of propylene with carbon monoxide CO and dihydrogen H₂ in the presence of a catalyst according to the present invention;
Figure 2 is representation of the ligand "cataCXium^{®}".

### DETAILED DESCRIPTION OF THE INVENTION

The present invention relates to a process for the hydroformylation of an aliphatic alkene with carbon monoxide CO and dihydrogen H₂ under catalytic conditions.

In particular, the present invention concerns a method for the hydroformylation of an aliphatic alkene with carbon monoxide CO and dihydrogen H₂, the method implementing a catalyst which comprises Palladium (0) complex of a first ligand L1 and Cobalt (0), the first ligand L1 being a monodentate phosphine.

The following description of the invention is limited to the sole hydroformylation of propylene under catalytic conditions.

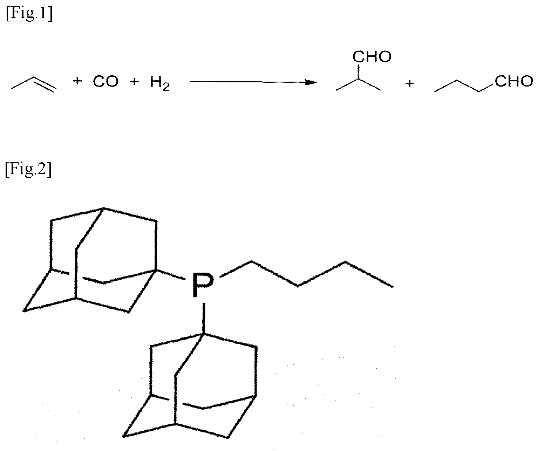

To this regard, figure 1 shows the chemical reaction associated with the hydroformylation of propylene with carbon monoxide CO and dihydrogen H₂ in the presence of a catalyst according to the present invention.

The process according to the terms of the present invention uses, in particular, a catalyst which comprises a blend of complex of palladium (0) having a first ligand L1 and cobalt (0).

The extension "(0)" used after palladium and cobalt indicates the oxidation state of the considered metal center.

The first ligand L1 can comprise at least one of the elements chosen among: tri-tert-butylphosphine of formula P(tBu)₃ (CAS : 13716-12-6), di(1-adamantyl)-n-butylphosphine, known as "cataCXium^{®}" (CAS : 321921-71-5), tert-butyldicyclohexylphosphine of formula P(tBu)Cy₂ (CAS : 93634-87-8), dicyclohexylphenylphosphine of formula PPhCy₂ (CAS : 6476-37-5), di-tert-butylphenylphosphine of formula PPh(tBu)₂ (CAS : 32673-25-9). The Cobalt (0) can form a complex with a second ligand L2, said second ligand L2 being a bidentate phosphin. Notably, the second ligand L2 comprises at least one of the elements chosen among: bis(dicyclohexylphosphino) methane (dcypm ; CAS : 137349-65-6), 1,2-bis(dicyclohexylphosphino) ethane (dcype ; CAS : 23743-26-2), 1,3-bis(dicyclohexylphosphino) propane (dcypp ; CAS : 103099-52-1), 1,4-bis(dicyclohexylphosphino) butane (dcypb ; CAS : 65038-36-0).

In a particular embodiment, the molar ratio of palladium (0) over cobalt (0) can be in the 1:4 to 1:1 range.

The method can be carried out in a non-aqueous solvent which comprises at least one of the elements selected from: anisole, toluene, 1,4-dioxane, dimethylacetamide, p-xylene, decahydronaphthalene, DMF, DMSO, α,α,α-trifluorotoluene, hexafluorobenzene, 1,2-dichlorobenzene, methyl benzoate.

According to one embodiment, said method is carried out in short-chain aldehydes as solvent. By "short-chain aldehydes", it is meant aldehydes containing a carbon chain equal or inferior to 5. Advantageously, said method is carried out in butanal or isobutanal.

According to one embodiment, said method is carried out without any external solvent.

The method can be carried out at a temperature of between 70 °C and 200 °C, advantageously in the 80 to 120 °C range.

Carbon monoxide CO and dihydrogen H₂ can be provided at a pressure up to 10 bar.

The aliphatic alkene can be provided at a pressure up to10 bar.

The following table 1 shows the catalytic activity (Readout 1 (M1 TOF) for different catalytic combinations implemented for the hydroformylation of propylene which yields isobutanal (B) and butanal (L). This table considers a combination of a complex of Metal 1 (M1) and metal 2 (M2). "M1 ligand" and "M2 ligand" correspond, respectively, to the first ligand L1 and the second ligand L2.

**Table 1:**

| | Combination of catalysts | | M1 Ligand | M2 Ligand | Ratio M1:M2 | Readout 1 (M1 TOF) | | Activity decay |
|---|---|---|---|---|---|---|---|---|
| | Metal 1 | (M1) 2 (M2) | | | | 0-2 hours | 2-6 hours | |
| 1 | Pd | Co | P tBu, | dcypp | 1:4 | 139 | 60 | 57% |
| 2 | Pd | Co | P tBu, | | 1:4 | 297 | 32 | 89% |
| 3 | Pd | | P tBu, | | | 0,03 | 0,1 | |
| 4 | | Co | | dcypp | | 19* | 14* | 26% |
| 5 | | Co | | | | 23* | 12* | 48% |
| δ | Pd | Co | P tBu₂ | dcypm | 1:4 | 104 | 55 | 47% |
| 7 | Pd | Co | P tBu₂ | dcypp | | 139 | 60 | 57% |
| 8 | Pd | Co | cataCXium | dcypb | 1:4 | 185 | 96 | 48% |
| 9 | Pd | Co | cataCXium | | 1:4 | 150 | 28 | 81% |
| 10 | Pd | Co | P tBuCy₂ | dcypb | 1:4 | 120 | 46 | 62% |
| 11 | Pd | Co | P PhCy₂ | dcypb | 1:4 | 107 | 41 | 62% |
| 12 | Pd | Co | P PhtBu₂ | dcypb | 1:4 | 257 | 38 | 85% |

| | | | | | | | | |
|---|---|---|---|---|---|---|---|---|
| * - TOF calculated on Co corresponds to about 4x higher values for Pd (for TOF of lines 4 and 5). Therefore, the TOF calculated on Co alone shall be multiplied by 4 before comparing it with the TOF calculated on Pd. | | | | | | | | |

The column "Ratio M1:M2" is associated with the molar ratio of metal 1 over metal 2, namely palladium (0) over cobalt (0).

The Readout 1 corresponds to the TOF (turnover frequency). This TOF is calculated for each sample on the basis of the Gas Chromatography (GC) analysis evaluating the content of butanal and isobutanal after the reaction of hydroformylation of propylene. The molar amounts of butanal and isobutanal are then added and the result is divided by the molar content of palladium or cobalt (TOF values in the table are calculated for Pd, unless otherwise specified, in case if it is not present in the sample). The resulting value of TON is then divided by the time in hours, providing the TOF.

GC measurements were calibrated on samples containing 2 mL of anisole with different amounts of butanal and isobutanal in 1: 1 ratio. Octane and dodecane stock solution in 1,4-dioxane was added to all samples (0,2 mL) and after vigorous mixing, 0,1 mL of such sample was subjected to GC. This stock solution was used for all analytical samples considered. In case of metal precipitation in the sample, it is being filtered after the addition of the standard to ensure that the ratio of soluble compounds remains the same.

Based on the retention times of four compounds of interest, a linear relationship was derived and was used to evaluate the molar content of aldehydes. A test sample with known amount of both aldehydes matched with the expected result. Due to the nature of the products, in the case of heavy derivative formation in longer experiments, some of them may overlap with standard peaks on the GC spectrum. If such situation arises, overlapping signal is ignored and only second standard is taken into consideration when calculating results. This can be easily seen by the difference between results calculated based on octane and dodecane standards.

The activity decay considers the loss of activity from the 0 to 2 hour range to the 2 to 6 hour range.

In the table 1, and in particular on lines 3 to 5, associated with a complex of palladium (0) alone, or to a complex of cobalt (0) alone, the activity in the 0 hour to 2 hour range is quite low and generally does not exceed 25 (which corresponds to an activity below 100 when multiplied by 4).

The sole consideration of a combination of a complex of Palladium (0) and a complex of Cobalt (0) strongly improves the activity.

Some specific combinations, and notably the combinations given in lines 1, 2, 6, 7, 8, 9, 10, 11, 12 exhibit strong activities and in particular TOF thereof above 100.

Some combinations, and notably the combination given in lines 6, 8 are associated with a quite low activity decay being below 50 %.

The catalyst combinations proposed in the present invention allow to consider hydroformylation of alkenes under green conditions.

Of course, the invention is not limited to the embodiments described and alternative embodiments may be made without departing from the scope of the invention as defined by the claims.

## Claims

1. A method for the hydroformylation of an aliphatic alkene with carbon monoxide and hydrogen, the method implementing a catalyst which comprises Palladium (0) complex of a first ligand L1 and Cobalt (0), the first ligand L1 being a monodentate phosphine.

2. The method according to claim 1, wherein the first ligand comprises at least one of the elements chosen among: tri-tert-butylphosphine of formula P(tBu)₃, di(1-adamantyl)-n-butylphosphine, known as "cataCXium^{®}", tert-butyldicyclohexylphosphine of formula P(tBu)Cy₂, dicyclohexylphenylphosphine of formula PPhCy₂, di-tert-butylphenylphosphine of formula PPh(tBu)₂.

3. The method according to claim 1 or claim 2, wherein the Cobalt (0) forms a complex with a second ligand L2, said second ligand L2 being a bidentate phosphine.

4. The method according to claim 3, wherein the second ligand L2 comprises at least one of the elements chosen among: bis(dicyclohexylphosphino) methane (dcypm), 1,2-bis(dicyclohexylphosphino) ethane (dcype), 1,3-bis(dicyclohexylphosphino) propane (dcypp), 1,4-bis(dicyclohexylphosphino) butane (dcypb).

5. The method according to any one of claim 1 to 4, wherein the molar ratio of palladium (0) over cobalt (0) is in the 1: 10 to 10: 1 range, advantageously in the 1:4 to 1: 1 range.

6. The method according the any one of claims 1 to 5, wherein the aliphatic alkene consists of propylene.

7. The method according to any one of claims 1 to 6, wherein said method is carried out in a non-aqueous solvent which comprises at least one of the elements selected from: anisole, toluene, 1,4-dioxane, dimethylacetamide, p-xylene, decahydronaphthalene, DMF, DMSO, α,α,α-trifluorotoluene, hexafluorobenzene, 1,2-dichlorobenzene, methyl benzoate.

8. The method according to any one of claims 1 to 6, wherein said method is carried out in short-chain aldehydes as solvent.

9. The method according to any one of claims 1 to 6, wherein said method is carried out without any external solvent.

10. The method according to any of claims 1 to 9, wherein said method is carried out at a temperature of between 70 °C and 200 °C, advantageously in the 80 to 120 °C range.

11. The method according to any of claims 1 to 10, wherein carbon monoxide and hydrogen are provided at a pressure up to 50 bar each, preferably up to 10 bar each.

12. The method according to any of claims 1 to 11, wherein the aliphatic alkene is provided at a pressure up to 10 bar.
